(19) Europäisches Patentamt / European Patent Office / Office européen des brevets

(11) **EP 4 142 696 B1**

(12) **EUROPEAN PATENT SPECIFICATION**

(45) Date of publication and mention of the grant of the patent:
**04.03.2026 Bulletin 2026/10**

(21) Application number: **21721562.3**

(22) Date of filing: **29.04.2021**

(51) International Patent Classification (IPC):
**A61K 9/16** (2006.01)    **A61K 31/366** (2006.01)
**A61K 9/14** (2006.01)

(52) Cooperative Patent Classification (CPC):
**A61K 9/1635; A61K 9/143; A61K 9/146; A61K 9/1611; A61K 9/1641; A61K 9/1652; A61K 31/366**

(86) International application number:
**PCT/EP2021/061310**

(87) International publication number:
**WO 2021/219808 (04.11.2021 Gazette 2021/44)**

(54) **SOLID FORMULATIONS OF CORALLOPYRONIN A**

FESTE FORMULIERUNGEN VON CORALLOPYRONIN A

FORMULATIONS SOLIDES DE CORALLOPYRONINE A

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.04.2020 EP 20172409**

(43) Date of publication of application:
**08.03.2023 Bulletin 2023/10**

(73) Proprietor: **Rheinische Friedrich-Wilhelms-Universität Bonn**
**53113 Bonn (DE)**

(72) Inventors:
- **WAGNER, Karl G.**
  **53121 Bonn (DE)**
- **KROME, Anna Katharina**
  **53129 Bonn (DE)**
- **HÖRAUF, Achim**
  **53125 Bonn (DE)**
- **KÖNIG, Gabriele M.**
  **53127 Bonn (DE)**
- **PFARR, Kenneth Michael**
  **53129 Bonn (DE)**
- **SCHIEFER, Andrea**
  **53639 Königswinter (DE)**
- **KEHRAUS, Stefan**
  **53347 Alfter (DE)**
- **HÜBNER, Marc Peter**
  **53340 Meckenheim (DE)**

(74) Representative: **Michalski Hüttermann & Partner Patentanwälte mbB**
**Kaistraße 16A**
**40221 Düsseldorf (DE)**

(56) References cited:
**EP-A1- 2 520 293**

- **YANBIN HUANG ET AL: "Fundamental aspects of solid dispersion technology for poorly soluble drugs", ACTA PHARMACEUTICA SINICA B, vol. 4, no. 1, February 2014 (2014-02-01), pages 18 - 25, XP055497465, ISSN: 2211-3835, DOI: 10.1016/ j.apsb.2013.11.001**

Remarks:
The file contains technical information submitted after the application was filed and not included in this specification

**Description**

The present invention relates to solid formulations of corallopyronin A.

Corallopyronin A is a natural compound produced by gliding myxobacteria, such as *Corallococcus coralloides.* Corallopyronin A also is denoted methyl N-[(E)-5-[6-hydroxy-5-[(2E,4E,9Z,13E)-8-hydroxy-2,5,9-trimethylpentadeca-2,4,9,13-tetraenoyl]-4-oxopyran-2-yl]hex-1-enyl]carbamate according to the IUPAC nomenclature. Corallopyronin A is known to provide antibacterial properties since 1985. EP 2 520 293 A1 further describes corallopyronin A being a compound usable in the treatment of filariasis.

However, corallopyronin A is a highly lipophilic compound and poorly soluble in water-based solvents. Since solubility is one of the important parameters for bioavailability and oral administration, there is a need of pharmaceutically suitable formulations of corallopyronin A. As the compound corallopyronin A has a semisolid, sticky and oily consistency that is not usable for tableting or filling into capsules or crystallization as a solid compound, and pharmaceutical formulations of corallopyronin A are not available so far.

Therefore, the objective underlying the present invention was to provide a formulation of corallopyronin A that is usable for the manufacture of orally administrable pharmaceutical drug products.

The problem is solved by a solid formulation comprising corallopyronin A. wherein the formulation comprises an amorphous solid dispersion of corallopyronin A embedded in a water-soluble polymer selected from the group of polyvinylpyrrolidone and polyvinylpyrrolidone/vinylacetate copolymer.

Surprisingly it was found that embedding corallopyronin A in a water-soluble polymer as claimed can provide solid formulations of corallopyronin A that are orally administrable. The solid formulations can be filled into capsules, pressed to tablets, and thus provide usual dosable and convenient oral dosage forms of corallopyronin A.

Solid formulations comprising an amorphous solid dispersion of corallopyronin A embedded in a water-soluble polymer as claimed provide advantageously stable formulations. The term "water-soluble" polymers denotes polymers that dissolve, disperse, or swell in water. The term "water-soluble" as used herein particularly also encompasses polymers with pH-dependent solubility, such as at pH 1 to 7, preferably at pH 1 to 5 and pH 5.5 to 7. Polymers with a pH-dependent solubility include poly (butyl methacrylate, 2-dimethyl aminoethyl methacrylate), polyvinyl acetate phthalate (PVAP) and hydroxypropylmethylcellulose acetate succinate (HPMCAS). Further polymers with a pH-dependent solubility include poly (methyl methacrylate, methacrylic acid) and poly (methacrylic acid ethyl acrylate).

The water-soluble polymer is selected from the group of polyvinylpyrrolidone (PVP) and polyvinylpyrrolidone/-vinylacetate copolymer (PVP/VA).

In embodiments, the water-soluble polymer comprises corallopyronin A in an amount in a range of $\geq 10$ weight% to $\leq 50$ weight%, preferably in an amount in a range of $\geq 15$ weight% to $\leq 40$ weight%, further preferred in an amount in a range of $\geq 20$ weight% to $\leq 30$ weight%, based on a weight of 100 weight% of the water-soluble polymer and corallopyronin A.

In preferred embodiments, the formulation comprises corallopyronin A embedded in polyvinylpyrrolidone. Particularly amorphous solid dispersions of corallopyronin A embedded in polyvinylpyrrolidone showed advantageous solubility and stability. In further preferred embodiments, the formulation comprises corallopyronin A embedded in polyvinylpyrrolidone/vinylacetate copolymer (PVP/VA).

Embedding corallopyronin A in a water-soluble polymer as claimed can provide orally administrable solid formulations of corallopyronin A. In preferred embodiments, the formulation is a solid oral formulation. In embodiments, the solid formulation is formulated into a capsule, a tablet, granules, pills, pellets, or micro-tablets.

A further aspect of the present invention relates to a pharmaceutical solid dosage form, comprising the solid formulation comprising corallopyronin A according to the invention. In embodiments, the solid dosage form is selected from a capsule, a tablet, granules, pills, pellets or micro-tablets. For the further description of the solid pharmaceutical formulation comprising corallopyronin A, reference is made to the description above.

A further aspect of the present invention relates to a process of manufacturing a solid formulation according to the invention, comprising the steps of:

a) dispersing, dissolving or otherwise introducing corallopyronin A into an organic solvent to form a liquid mixture,
b) selecting a water-soluble polymer from the group of polyvinylpyrrolidone and polyvinylpyrrolidone/vinylacetate copolymer,
c) admixing the liquid mixture of step a) and the water-soluble polymer of step b), and
d) optionally removing excess solvent from the mixture obtained in step c) to form a solid formulation.

The solid formulation can be manufactured with little effort and standard equipment and thus is useful for preclinical and clinical development of pharmaceutical ingredients. Also industrial processing is possible in energy and cost effective, particularly compared to the manufacture of amorphous solid dispersions.

The solvent may be an organic solvent selected from ethanol, methanol, isopropanol, dichloromethane, acetone, tert-butanol, propylene carbonate, a C6 to C12 triglyceride, preferably a C8 or C10 triglyceride, a polymer which is liquid at ambient temperature such as a polyethylene glycol (PEG), preferably selected from PEG 400, PEG 300 and PEG 200, or mixtures thereof. The term "C6 to C12" triglycerides" refers to fatty acids that have a chain length of 6-12 carbon atoms.

The solid formulation may be orally administered. It is however preferred to further formulate the solid formulation into a usual pharmaceutical solid dosage form, such as a capsule, granules or a tablet. In embodiments of the process, in a further step e) the solid formulation is formed into a tablet, granules, pills, pellets or micro-tablets, preferably into capsules, granules or tablets. In embodiments, the process thus is a process for manufacturing capsules, granules or tablets comprising the solid formulation.

For manufacturing capsules, tablets or granules, the solid formulation may be filled into capsules, mixed with tableting additives and compressed to tablets, or wet or dry granulation can be applied to form granules, respectively. These methods are standard procedures known to a person skilled in the art and for example may be performed using usual tableting machines, capsule filling machines or suitable devices for wet granulation, e.g. high shear mixers, fluidized air granulators, or dry granulation, e.g. roller compactors. Preferably, the solid formulation is filled into capsules or mixed with tableting additives and compressed to tablets.

In a preferred embodiment, a solid formulation comprising corallopyronin A embedded in a water-soluble polymer as claimed is manufactured, the process comprising the steps of:

a) dispersing, dissolving or otherwise introducing corallopyronin A in an organic solvent to form a liquid mixture,
b) selecting a water-soluble polymer selected from the group of polyvinylpyrrolidone and polyvinylpyrrolidone/vinylacetate copolymer,
c) admixing the liquid mixture of step a) and the water-soluble polymer of step b), and
d) removing excess solvent from the mixture obtained in step c) to form a solid formulation.

In preferred embodiments for manufacturing a solid formulation comprising corallopyronin A embedded in a water-soluble polymer as claimed, the solvent is an organic solvent selected from ethanol, methanol, isopropanol, dichloromethane, acetone or tert-butanol. A preferred organic solvent is ethanol. Ethanol is preferred due to its low toxic potential. Advantageously, high amounts of corallopyronin A can be dissolved in ethanol, such as 100 mg/mL.

The dispersing, dissolving or otherwise introducing in step a) may be performed at a temperature in a range from ≥ 10 °C to ≤ 50 °C, preferably in a range from ≥ 15 °C to ≤ 25 °C, and/or may be performed using ultrasonic dissolving, vortexing, or mixing using magnetic mixers or blade agitators.

The water-soluble polymer is selected from the group of polyvinylpyrrolidone (PVP) and polyvinylpyrrolidone/-vinylacetate copolymer (PVP/VA).

The water-soluble polymer is selected in step b) and in step c) may be dispersed, dissolved or otherwise introduced into the liquid mixture of corallopyronin A in the organic solvent of step a). In other embodiments, in step b) the water-soluble polymer may be dispersed or dissolved in water, an organic solvent, or a mixture thereof. In these embodiments, in step c) liquid mixtures of step a) and of step b) may be mixed and may result in a mixture of organic solvents or organic solvent and water.

In step d) excess solvent is removed from the mixture to form a solid oral formulation. The solvent may for example be removed by evaporating, such as by drying the formulation at ambient temperature or by using vacuum. The solvent or solvent mixture also may be removed by spray drying, freeze drying or spray freeze drying the mixture of step c) whereby also a solid oral formulation comprising corallopyronin A embedded in the water-soluble polymer is obtained. The evaporating or spray drying may be performed using standard equipment such as usual rotary evaporators and spray dryers.

A further aspect of the present invention relates to a solid formulation comprising corallopyronin A or a pharmaceutical solid dosage form obtained by the process according to the invention. For the description of the solid formulation and the pharmaceutical solid dosage forms, reference is made to the description above. Preferred pharmaceutical solid dosage forms are selected from a capsule, a tablet, granules, pills, pellets or micro-tablets.

Unless otherwise defined, the technical and scientific terms used herein have the same meaning as commonly understood by one of ordinary skill in the art to which this invention belongs.

The Examples, which follow serve to illustrate the invention in more detail but do not constitute a limitation thereof.

The figures show:

Figure 1    The biphasic dissolution profile of the organic phase of the amorphous formulation of corallopyronin A (CorA) embedded in polyvinylpyrrolidone (PVP) compared to pure corallopyronin A.

Figure 2    The biphasic dissolution profile of corallopyronin A embedded in polyvinylpyrrolidone.

Figure 3    The plasma concentration time profile of the liquid formulation and the solid formulation of corallopyronin A (CorA) embedded in polyvinylpyrrolidone *in vivo*.

Figure 4    The biphasic dissolution profile of an amorphous formulation of corallopyronin A in polyvinylpyrrolidone/ vinylacetate copolymer.

Figure 5    The biphasic dissolution profiles of an amorphous formulation of corallopyronin A embedded in polyethylene glycol.

Figure 6    The biphasic dissolution profiles of an amorphous formulation of corallopyronin A embedded in hydroxypropyl cellulose.

Figure 7    The biphasic dissolution profiles of the neat corallopyronin A (CorA) compound.

Figure 8    The plasma concentration time profile of corallopyronin A embedded in polyvinylpyrrolidone (CorA-PVP-ASD) or in polyvinylpyrrolidone / vinylacetate copolymer (CorA-PVP/VA-ASD) compared to neat corallopyronin A (CorA).

Reference

Example 1: Manufacture of an amorphous corallopyronin A formulation by polymer film casting using polyethylene glycol

0.1501 g Corallopyronin A (produced by the Myxobacterium *Corallococcus coralloides* B035; Helmholtz Centre for Infection Research, Dept. Microbial Drugs, Braunschweig) was weighted in a beaker and 2 ml ethanol were added. Thereafter the beaker was covered with parafilm and placed on an ultrasonic bath (Bandelin, Sonorex Digitec). The temperature of the bath was held to < 25 °C by replacing the water every 10 min. After 20 min the corallopyronin A was fully dissolved. In a further beaker 0.614 g polyethylene glycol (PEG 20000, Roth) was weighted and 10.825 mL ethanol and 0.675 mL water were added to the beaker. The beaker was covered with parafilm and placed on the ultrasonic bath for 60 min. The temperature of the bath was held to < 25 °C by replacing the water every 10 min. After 60 min the polymer was fully dissolved. Both solutions were mixed together with a metal spatula and placed on the ultrasonic bath for another 5 min. After mixing the mixture was transferred into a 250 mL perfluoroxy alkane (PFA) round-bottom flask and dried at 25 °C for 24 hours at a vacuum of 600 - 0 mbar and 50 rpm on a rotary evaporator (Laborata 4000 efficient, Heidolph). Solvent evaporation yielded an amorphous solid formulation of corallopyronin A embedded in polyethylene glycol. The drug load was calculated to about 20 weight%.

Reference

Example 2: Manufacture of an amorphous corallopyronin A formulation by polymer film casting using hydroxypropyl cellulose

0.1539 g Corallopyronin A (produced by the Myxobacterium *Corallococcus coralloides* B035; Helmholtz Centre for Infection Research, Dept. Microbial Drugs, Braunschweig) was weighted in a beaker and 2 mL ethanol were added. Thereafter, the beaker was covered with parafilm and placed on an ultrasonic bath (Bandelin, Sonorex Digitec). The temperature of the bath was held to ≤ 25 °C by replacing the water every 10 min. After 20 min the corallopyronin A was fully dissolved. In a further beaker 0.6001 g hydroxypropyl cellulose super super fine (HPC SSL, Nisso) was weighted and 11.5 mL ethanol was added to the beaker. The beaker was covered with parafilm and placed on the ultrasonic bath. The temperature of the bath was held to ≤ 25 °C by replacing the water every 10 min. After 40 min the polymer was fully dissolved. Both solutions were mixed together with a metal spatula and placed on the ultrasonic bath for another 5 min. After mixing the mixture was transferred into a 250 mL perfluoroxy alkane (PFA) round-bottom flask and dried at 25 °C for 24 hours at a vacuum of 600 - 0 mbar and 50 rpm on a rotary evaporator (Laborata 4000 efficient, Heidolph). Solvent evaporation yielded an amorphous solid formulation of corallopyronin A embedded in hydroxypropyl cellulose. The drug load was calculated to 20 weight%.

Example 3: Manufacture of an amorphous formulation of corallopyronin A embedded in polyvinylpyrrolidone by spray drying

1.6022 g Corallopyronin A (produced by the Myxobacterium *Corallococcus coralloides* B035; Helmholtz Centre for Infection Research, Dept. Microbial Drugs, Braunschweig) was weighted in a beaker and 16.0 mL ethanol was added. Thereafter, the beaker was covered with parafilm and placed on an ultrasonic bath (Bandelin, Sonorex Digitec). The temperature of the bath was held to < 25 °C by replacing the water every 10 min. After 40 min the corallopyronin A was fully dissolved. In a further beaker 6.4237 g polyvinylpyrrolidone (PVP-K30 LP, BASF) was weighted in and 51.0 mL ethanol was added. The beaker was covered with parafilm and placed on the ultrasonic bath at 25 °C. After 5 min the polymer was dissolved. Both solutions were mixed together with a metal spatula and placed on the ultrasonic bath for another 5 min. After mixing, the pipe of the spray dryer (B-290; BÜCHI) was connected to the solution and the spray drying process started with an inlet temperature of 85 °C, an outlet temperature of 59 °C, aspirator 100% and pump running at 20%. Nitrogen was used as drying gas and the flow rate was set to 5.6 mL/min. Spray drying yielded an amorphous solid formulation of corallopyronin A embedded in polyvinylpyrrolidone. The drug load was calculated to about 20 weight%.

Example 4: Manufacture of an amorphous formulation of corallopyronin A embedded in polyvinylpyrrolidone/vinylacetate copolymer by spray drying

1.6008 g Corallopyronin A (produced by the Myxobacterium *Corallococcus coralloides* B035; Helmholtz Centre for Infection Research, Dept. Microbial Drugs, Braunschweig) was weighted in a beaker and 16 mL ethanol were added. Thereafter, the beaker was covered with parafilm and placed on an ultrasonic bath (Bandelin, Sonorex Digitec). The temperature of the bath was held to ≤ 25 °C by replacing the water every 10 min. After 40 min the corallopyronin A was fully dissolved. In a further beaker 6.4032 g polyvinylpyrrolidone/vinylacetate copolymer (Kollidon VA 64; BASF) was weighted and 51.0 mL ethanol was added. The beaker was covered with parafilm and placed on the ultrasonic bath at 25 °C. After 5 min the polymer was dissolved. Both solutions were mixed together with a metal spatula and placed on the ultrasonic bath for another 5 min. After mixing the pipe of the spray dryer (B-290; BÜCHI) was connected to the solution and the spray drying process started with an inlet temperature of 85 °C, an outlet temperature of 59 °C, aspirator 100% and pump running at 20%. Nitrogen was used as drying gas and the flow rate was set to 4.5 mL/min. Spray drying yielded an amorphous solid formulation of corallopyronin A embedded in polyvinylpyrrolidone/vinylacetate copolymer. The drug load was calculated to about 20 weight%.

Reference

Example 5: Manufacture of a corallopyronin A formulation by adsorption to mesoporous silica

0.1012 g Corallopyronin A (produced by the Myxobacterium *Corallococcus coralloides* B035; Helmholtz Centre for Infection Research, Dept. Microbial Drugs, Braunschweig) was weighted in a 3 mL glass vial and 300 μl ethanol was added. Thereafter, the glass vial was covered with parafilm and placed on an ultrasonic bath (Bandelin, Sonorex Digitec). The temperature of the bath was held to ≤ 25 °C by replacing the water every 10 min. After 20 min the corallopyronin A was fully dissolved. Thereafter, 0.1021 g of mesoporous silica (Syloid XRD 3050, Grace GmbH, Worms) was added to the glass vial and the mixture was mixed with a metal spatula and dried for 24 h under the fume hood at 20 °C. Drying yielded a solid formulation of corallopyronin A loaded onto mesoporous silica. The drug load was calculated to about 50 weight%.

Example 6: Pharmacological testing of the amorphous formulation of corallopyronin A embedded in polyvinylpyrrolidone

The amorphous formulation of corallopyronin A embedded in polyvinylpyrrolidone by spray drying as prepared in example 3 was tested for dissolution performance, pharmacokinetics and stability.

6.1 Biphasic dissolution tests

Biphasic release is a process in which, in addition to solubility in an aqueous medium, adsorption into the intestinal wall is demonstrated using an organic phase. The transition to the organic phase can be understood as an absorption of active substances into the blood. The biphasic release experiments were investigated using a fully automated biphasic dissolution apparatus (BiPHa+) as described in A. Denninger et al., Pharmaceutics 2020, 12, 237.
To simulate the gastrointestinal properties to obtain *in vivo* predictive results, the pH-profile, bile salt concentration and gastrointestinal passage time were adjusted in the aqueous phase to mimic human gut conditions. Additionally, an organic phase above the aqueous phase imitates the fraction absorbed from the gut. An organic phase of 1-decanol above the aqueous phase imitated the fraction absorbed from the gut. As a test model, the profile of a person without prior food intake was chosen. Therefore, FaSSIF-V2 medium, which represents the bile salts concentration of a fasted human was used. FaSSIF-V2 medium is a mixture of a phosphate and a citrate buffer system, which facilitates comparable *in vivo* buffer capacities. To generate an in-situ biorelevant aqueous medium for the fasted state, biorelevant surfactants, namely sodium-taurocholate (3 mM) and lecithin (0.2 mM), were added.
Prior to the start of the experiments, both phases, 1-decanol and the acidic aqueous phase, were saturated with each other. First, about 10 mg of the API corallopyronin A in the respective formulation was added to 50 mL HCl (0.1 M), simulating the stomach. During the first thirty minutes the formulation disintegrated / dispersed in 50 mL of 0.1N HCl.
After 30 min FaSSIF-V2 concentrate (sodium-taurocholate and lecithin) was added simultaneously to the addition of citrate-phosphate buffer (tri-potassium phosphate and potassium citrate) resulting in a first pH-shift from pH 1 to pH 5.5, simulating the duodenum. Thereafter, 50 mL decanol was added. The last pH-shift from 5.5 to 6.8 after 90 minutes was gradually adjusted by adding more citrate-phosphate buffer, representing the jejunum and ileum (final concentrations: 3 mM sodium-taurocholate, 0.2 mM lecithin, 525 mM tri-potassium phosphate and 225 M potassium citrate). The adjusted buffers were titrated by 0.1 M NaOH and 0.1 M HCl (pH 5.5-6.8). The whole dissolution took 4.5 hours.

The concentration profiles of the aqueous and organic phase were measured online continuously with an Agilent 8454 UV-Vis spectrometer (Waldbronn, Germany) and quantified on the compound-specific wavelength. Three independent dissolution tests were performed.

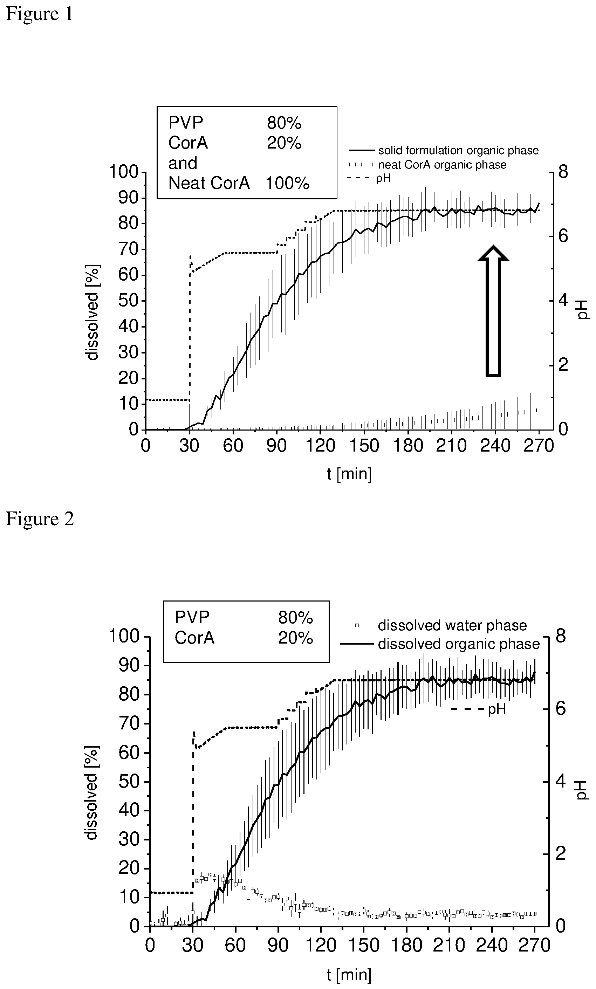

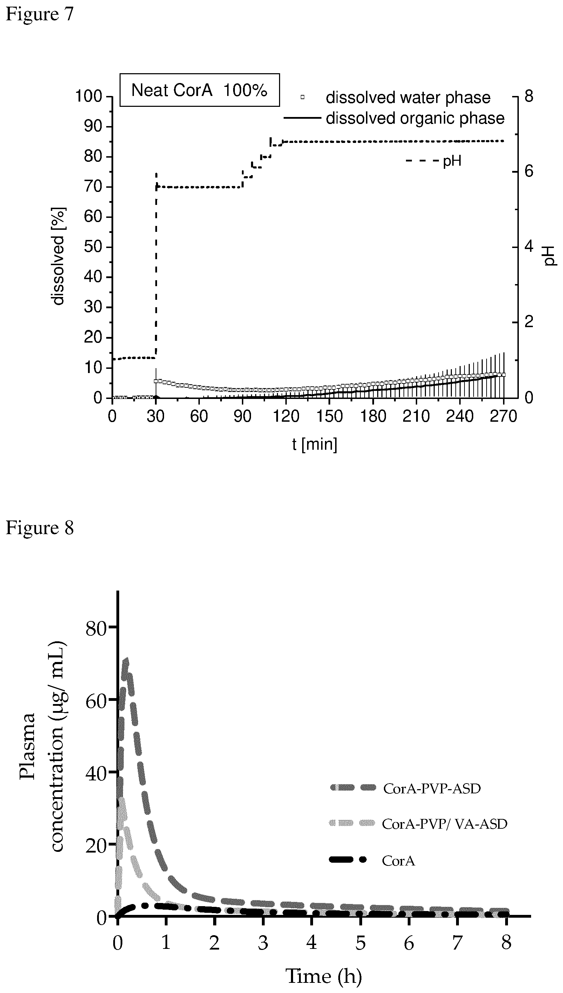

Figure 1 shows the pH profile and illustrates the biphasic dissolution profile of the amorphous formulation of corallopyronin A embedded in polyvinylpyrrolidone as prepared in example 3 compared to neat corallopyronin A. As can be taken from figure 1, the amorphous formulation of corallopyronin A provided 87.6 % predicted absorption. The formulation thus provides very good *in vitro* performance. Compared to the solubility of the neat compound corallopyronin A, as described in comparative example 10, the biphasic dissolution model for human absorption shows a more than 10 fold increase of fraction absorbed due to solubility enhanced amorphous solid formulation. Individual dissolution graphs of corallopyronin A embedded in polyvinylpyrrolidone and of the pure corallopyronin A compound are provided in figures 2 and 7, respectively.

6.2 Pharmacokinetic analysis

Pharmacokinetic analysis was performed *in vivo* in fed BALB/c female mice (n=4) (Study DZIF11). The solid formulation was suspended right before administration in PBS (phosphate buffered saline; pH=7.4) and was administered via gavage (36 mg/kg CorA, 10 mL/kg). Blood samples from the vena facialis were collected after 5, 10, 15, 30, 180, and 480 min. The samples were centrifuges for 10 min at 4 °C and 15000 g. The generated plasma was mixed in a ration of 1:3 with ice-cold acetonitrile (Bernd Kraft GmbH, Duisburg). The mixture was vortexed for 10 sec and centrifuged for 25 min at 4 °C and 11600 g. The HPLC measurements were performed on a Waters Alliance e2695 separation module connected to a waters 2998 PDA detector. A waters XBridge® Shield RP$_{18}$, 3.5 μm, 2.1x100 mm, 130 A column was used. For the gradient method starting from 70% A/ 30% B to 20% A/ 80 % B stepwise within 30 min, the mobile phases A (acetonitrile/-water (Bernd Kraft GmbH, Duisburg) 5/95 with 5 mM ammonium acetate (Merck KGaA, Darmstadt) and 40 μL acetic acid (Sigma-Aldrich Chemie GmbH, Steinheim) per Liter) and B (acetonitrile/water 95/5 with 5 mM ammonium acetate and 40 μL acetic acid per Liter) enabled to quantitate corallopyronin A precisely at 300 nm at a flow rate of 0.3 mL/min and a column temperature of 30 °C. The samples were quantitated via external reference standard. The content of the corallopyronin A reference material was analyzed by $^1$H-NMR.

Plasma concentration-time profiles were generated and pharmacokinetic parameters calculated.

To calculate the absolute bioavailability of the oral formulation, an intravenous (IV) profile was conducted. Therefore, corallopyronin A (CorA) was prepared in a liquid formulation (not according to the claims) (propylene glycol (20%) (Caesar & Lorenz GmbH, Hilden); Kolliphor® HS-15 (20 %) (Sigma-Aldrich Chemie GmbH, Taufkirchen); PBS pH 7.4 (60 %)). The prepared solution was then administered intravenously (36 mg/kg CorA, 5 mL/kg) in the tail vein. The following table 1 shows the results of the pharmacokinetic analysis.

Table 1: Results of the pharmacokinetic analysis

| pharmacokinetic analysis (median values) | IV liquid CorA formulation (PG; K-HS15; PBS) (36 mg/kg) | PO solid CorA formulation (PVP) (36 mg/kg) |
|---|---|---|
| AUC (0-8h) [μg*h/mL] | 115.5 | 67.8 |
| AUC (0-inf) [μg*h/mL] | 127.7 | 75.9 |
| c $_{max}$ [μg/mL] | 119.6 | 64.3 |
| t $_{max}$ [min] | 5* *first measured value | 10 |
| Fabs [%] | 100 | 59 |

Table 1 shows the pharmacokinetic parameters after the IV administration of the liquid formulation and the PO administration of the solid formulation. The area under the concentration-time over all time was calculated for the IV application as 127.7 μg*h/mL (median value) and for the PO application as 75.9 μg*h/mL (median value). Based on the AUC results, the absolute bioavailability after the PO was calculated to be 59% ($F_{abs}$=(AUC$_{(0-inf)}$ IV/ AUC$_{(0-inf)}$ PO)* 100), demonstrating a high oral bioavailability in mice due to the enhanced solubility formulation principle.

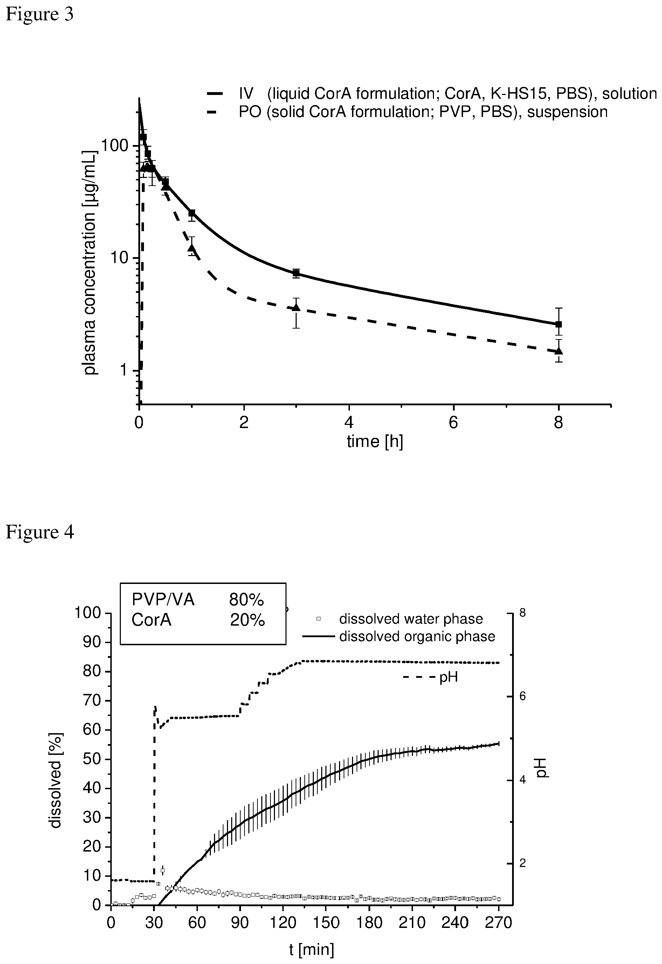

Figure 3 illustrates the plasma concentration-time profile of the IV administration of the CorA solution and the PO administration of the solid formulation *in vivo.* The fast course and high measured values of the PO curve demonstrate the solubility enhancement of the formulation principle since a fast and high oral absorption of an active pharmaceutical ingredient is only possible when the API is dissolved and transported via passive or active transport into the blood circulation. Due to the lipophilic character of corallopyronin A a sufficient absorption through membranes was predicted in combination with the solubility enhanced formulation principle a sufficient oral bioavailability in mice was demonstrated.

6.3 Stability analysis

The stability of the spray dried amorphous formulation of corallopyronin A embedded in polyvinylpyrrolidone was analyzed after storing samples in drying cabinets at 25 °C / 60% relative humidity (RH) and under accelerated conditions at 40 °C/75% RH for 7, 14, 28 days and 3 months. The samples were stored in closed twist-off glass vials under nitrogen and in the presence of silica desiccant. The content was analyzed by High-Performance Liquid Chromatography with Diode-Array Detection (HPLC-DAD). The samples were, therefore, dissolved in acetonitrile (Bernd Kraft GmbH, Duisburg). The HPLC measurements were performed on a Waters Alliance e2695 separation module connected to a waters 2998 PDA detector. A waters XBridge® Shield RP$_{18}$, 3.5 $\mu$m, 2.1x100 mm, 130 A column was used. For the gradient method starting from 70% A/ 30% B to 20% A/ 80 % B stepwise within 30 min, the mobile phases A (acetonitrile/water (Bernd Kraft GmbH, Duisburg) 5/95 with 5 mM ammonium acetate (Merck KGaA, Darmstadt) and 40 $\mu$L acetic acid (Sigma-Aldrich Chemie GmbH, Steinheim) per Liter) and B (acetonitrile/water 95/5 with 5 mM ammonium acetate and 40 $\mu$L acetic acid per liter) enabled to quantitate corallopyronin A precisely at 300 nm at a flow rate of 0.3 mL/min and a column temperature of 30 °C. The samples were quantitated via external reference standard. The content of the corallopyronin A reference material was analyzed by $^1$H-NMR. The following table 2 summarizes the results of the stability analysis.

Table 2: Results of the stability analysis

| Storace Duration | Storace Condition | CorA Content [%] |
|---|---|---|
| 0 | | 100 |
| 7 days | 25 °C; 60% RH | 100 |
| 14 days | 25 °C; 60% RH | 100 |
| 28 days | 25 °C; 60% RH | 98 |
| 3 months | 25 °C; 60% RH | 98 |
| 7 days | 40 °C; 75% RH | 98 |
| 14 days | 40°C; 75% RH | 97 |
| 28 days | 40 °C; 75% RH | 93 |
| 3 months | 40 °C; 75% RH | 83 |

The results of table 2 show that the spray dried amorphous formulation of corallopyronin A embedded in polyvinylpyrrolidone showed good long term stability at 25 °C, and also provided sufficient stability under conditions of accelerated decay. Compared to the highly instable compound corallopyronin A as shown in example 10.2, the spray dried formulation with polyvinylpyrrolidone provides a considerable stability enhancement. This shows that formulations embedding corallopyronin A in water-soluble polymers such as polyvinylpyrrolidone provides solubility and stability enhancements.

Example 7: Pharmacological testing of the amorphous formulation of corallopyronin A embedded in polyvinylpyrrolidone/vinylacetate copolymer

7.1 Biphasic dissolution tests

The amorphous formulation of corallopyronin A embedded in polyvinylpyrrolidone/ vinylacetate copolymer by spray drying as prepared in example 4 was tested for dissolution performance as described in example 6.1.
The figure 4 shows the pH profile and illustrates the biphasic dissolution profile of the amorphous formulation of corallopyronin A in polyvinylpyrrolidone/ vinylacetate copolymer. As can be taken from figure 4, the formulation provided 55.4 % predicted absorption. The formulation thus provides good *in vitro* performance.

7.2 Stability analysis

The stability of the amorphous formulation of corallopyronin A in polyvinylpyrrolidone/ vinylacetate copolymer was tested as described in example 6.3. The following table 3 summarizes the results of the stability analysis.

Table 3: Results of the stability analysis

| Storace Duration | Storace Condition | CorA Content [%] |
|---|---|---|
| 0 | | 100 |
| 7 days | 25 °C; 60% RH | 100 |
| 14 days | 25 °C; 60% RH | 100 |
| 28 days | 25 °C; 60% RH | 99 |
| 3 months | 25 °C; 60% RH | 98 |
| 7 days | 40°C; 75% RH | 100 |
| 14 days | 40 °C; 75% RH | 99 |
| 28 days | 40 °C; 75% RH | 98 |
| 3 months | 40 °C; 75% RH | 88 |

The results of table 3 show that the spray dried amorphous formulation of corallopyronin A embedded in polyvinylpyrrolidone/ vinylacetate copolymer (PVP/VA) showed good long term stability at 25 °C, and also provided sufficient stability under conditions of accelerated decay. This shows that also a stability enhancement was achieved by embedding the corallopyronin A in PVP/VA. This shows that also formulations embedding corallopyronin A in PVP/VA provide solubility and stability enhancements.

Reference

Example 8: Biphasic dissolution test of the amorphous formulation of corallopyronin A embedded in polyethylene glycol

The amorphous formulation of corallopyronin A embedded in polyethylene glycol by film casting as prepared in reference example 1 was tested for dissolution performance as described in example 6.1.

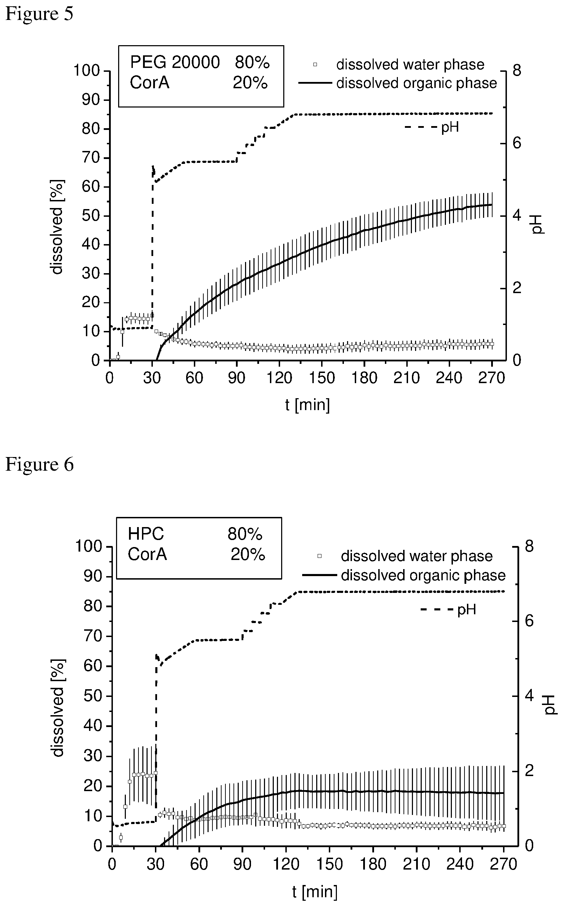

Figure 5 shows the pH profile and illustrates the biphasic dissolution profile of the amorphous formulation of corallopyronin A embedded in polyethylene glycol. As can be taken from figure 5, the formulation provided 53.9 % predicted absorption. The formulation thus provides good *in vitro* performance.

Reference

Example 9: Biphasic dissolution test of the amorphous formulation of corallopyronin A embedded in hydroxypropyl cellulose

The amorphous formulation of corallopyronin A embedded in hydroxypropyl cellulose by film casting as prepared in reference example 2 was tested for dissolution performance as described in example 6.1.
Figure 6 shows the pH profile and illustrates the biphasic dissolution profile of the amorphous formulation of corallopyronin A embedded in hydroxypropyl cellulose. As can be taken from figure 6, the formulation provided 17.8 % predicted absorption.

Comparative example 10: Pharmacological testing of pure corallopyronin A compound

10.1 Biphasic dissolution tests

Corallopyronin A was tested for dissolution performance as described in example 6.1. Figure 7 shows the pH profile and the biphasic dissolution profile of neat corallopyronin A compound (CorA). As can be taken from figure 7, the pure substance only provided 7.7 % predicted absorption.

10.2 Stability analysis

The stability of neat corallopyronin A compound was tested as described in example 6.3. The following table 4 summarises the results of the stability analysis.

Table 4: Results of the stability analysis of Corallopyronin A

| Storace Duration | Storace Condition | CorA Content [%] |
|---|---|---|
| 0 | | 99 |
| 7 days | 25 °C; 60% RH | 85 |
| 14 days | 25 °C; 60% RH | 76 |
| 28 days | 25 °C; 60% RH | 63 |
| 3 months | 25 °C; 60% RH | 39 |
| 7 days | 40 °C; 75% RH | 47 |
| 14 days | 40 °C; 75% RH | 31 |
| 28 days | 40°C; 75% RH | 17 |
| 3 months | 40 °C; 75% RH | 6 |

The results of table 4 show that the corallopyronin A content decreases fast at 25 °C and under conditions of 40 °C; after 7 days the corallopyronin A content at 25 °C falls below 90% and after 3 months below 40%; at accelerated conditions (40°C) the collopyronin A content falls below 50 % within 7 days and below 10 % within 3 months.

Reference

Example 11: Manufacture of amorphous solid dispersions of corallopyronin A embedded in various polymers

Amorphous solid dispersion formulations were prepared via film casting or spray drying as described in Reference Examples and 2, and Examples 3 and 4, respectively, with the exception that the polymers summarised in table 5 were used.

Table 5: Polymers used for the amorphous solid dispersion formulations

| Polymer | Supplier |
|---|---|
| Eudragit® E100 | Evonik Industries AG (Essen, Germany) |
| Eudragit® L100-55 | Evonik Industries AG (Essen, Germany) |
| AquaSolve™ HPMCAS | Ashland Industries Deutschland GmbH, (Düsseldorf, Deutschland) |
| HPMCP 55 | Shin-Etsu (Tokyo, Japan) |
| Phthalavin® PVAP | Colorcon GmbH (Idstein, Germany) |
| Soluplus® | BASF SE (Ludwigshafen, Germany |
| Kolliphor® P407 (Poloxamer) | BASF SE (Ludwigshafen, Germany |

Spray drying and film casting yielded amorphous solid dispersions of corallopyronin A embedded in the respective polymer. The following table 6 summarises the formulation technique and composition of the corallopyronin A amorphous solid dispersion (CorA-ASD) formulations.

Table 6: Formulation technique and composition of the CorA-ASD formulations (not according to the claims)

| active pharmaceutical ingredient (API) | Amount CorA (mg) | Polymer | Amount polymer (mg) | Solvent | Formulation Technique |
|---|---|---|---|---|---|
| CorA | 150 | Soluplus® | 601 | ethanol | film casting |
| CorA | 148 | Eudragit® E100 | 593 | ethanol | film casting |
| CorA | 151 | PVAP | 609 | methanol | film casting |
| CorA | 148 | Eudragit® L100-55 | 558 | ethanol | film casting |
| CorA | 149 | HPMCP | 602 | acetone | film casting |

(continued)

| active pharmaceutical ingredient (API) | Amount CorA (mg) | Polymer | Amount polymer (mg) | Solvent | Formulation Technique |
|---|---|---|---|---|---|
| CorA | 101 | Poloxamer P407 | 404 | ethanol | film casting |
| CorA | 149 | HPMCAS | 600 | dichlormethane/ methanol | film casting |

Reference

Example 12: Biphasic dissolution test of the amorphous solid dispersions of corallopyronin A embedded in various polymers

The performance of the formulations which were manufactured as described in Reference Example 11 compared to neat corallopyronin A were evaluated via the *in vivo* predictive *in vitro* biphasic dissolution apparatus BiPHa+ as described in Example 6.1.

The following table 7 summarises the Biphasic dissolution results compared to the results for neat corallopyronin A (CorA) as determined in comparative example 10. The term neat corallopyronin A (CorA) refers to the pure amorphous CorA material, which was produced by Myxobacterium *Corallococcus coralloides* B035 (Helmholtz Centre for Infection Research, Dept. Microbial Drugs, Braunschweig).

Table 7: Biphasic dissolution results in the organic phase of neat CorA and CorA-ASD formulations (not according to the claims)

| API | Polymer | Maximum Partitioned Drug in the Organic Phase (%) |
|---|---|---|
| CorA | / | 8 |
| CorA | Soluplus® | 23 |
| CorA | Eudragit® E100 | 43 |
| CorA | PVAP | 45 |
| CorA | Eudragit® L100-55 | 50 |
| CorA | HPMCP | 71 |
| CorA | Poloxamer P407 | 71 |
| CorA | HPMCAS | 82 |

As can be seen in table 7, for all formulations an increased amount partitioned into the organic phase. The organic phase *in vitro* simulates fasted human gut conditions, being a surrogate parameter for the fraction absorbed *in vivo*. The results predict for all CorA formulations an increased fraction to be absorbed *in vivo* and therefore an increased bioavailability compared to neat CorA.

Of neat CorA only 8% partitioned into the organic phase. In comparison, when CorA was embedded in a polymer of table 5 an increase of the partitioned amount was detected. If over the 4.5 h dissolution process in total at least 18% partitioned into the organic phase, such as when CorA was embedded into HPC as shown in reference example 9, this corresponds to a 2-fold increase. Under *in vivo* conditions an increase of 50% means that only 50% of the dosage would be needed, which represents a major advantage for the patient due to a lower risk of side effects and also a great economical advantage. By embedding CorA in Soluplus® an increase to 23% was achieved. When CorA was embedded in the polymers Eudragit® E100, PVP/VA, Eudragit® L100-55 or PEG 20000 an increase of 5-7fold was achieved. The best *in vitro* performance regarding the dissolution and solubility increasement were achieved when CorA was embedded in HPMCP, Poloxamer P407, HPMCAS or PVP, where an increase of 9-10 fold was achieved.

The biphasic dissolution results demonstrated a major benefit regarding the simulated fraction absorbed *in vitro*, which is considered predictive for results under *in vivo* conditions. An increase in bioavailability *in vivo* is, therefore, highly likely.

Example 13: Pharmacokinetic study of formulation of amorphous solid dispersion of corallopyronin A embedded in polyvinylpyrrolidone/vinylacetate copolymer

The formulation of corallopyronin A embedded in polyvinylpyrrolidone/vinylacetate copolymer was further evaluated in a pharmacokinetic study to evaluate the performance *in vivo* in BALB/c mice compared to neat corallopyronin A as described in Example 6.2.

A liquid formulation of CorA (not according to the claims) was prepared comprising propylene glycol (20%, Carl Roth GmbH +. Co. KG), Kolliphor® HS-15 (20%), and PBS pH 7.4 (60%). This CorA-solution was administered intravenously (36 mg/kg CorA, 5 mL/kg) in the tail vein to establish an intravenous (IV) pharmacokinetic profile.

Absolute bioavailability of the oral formulation was calculated according to the following Equation (1):

$$F_{abs} (\%) = 100 * \frac{AUC_{(0-inf)} PO}{AUC_{(0-inf)} IV}$$

$$(1),$$

wherein

$F_{abs}$ = absolute bioavailability (%),
$AUC_{(0-inf)}$ IV = area under the curve after intravenous administration,
$AUC_{(0-inf)}$ PO = area under the curve after per oral administration.

The results are illustrated in figure 8 and in table 8. Table 8 shows the oral bioavailability of oral administration (PO) of CorA-PVP/VA-ASD formulation and neat corallopyronin A (CorA) compared to intravenous (IV) administration set to 100%.

Table 8. Increased oral bioavailability in BALB/c mice after the oral administration of CorA-PVP-ASD formulation compared to neat CorA.

| API | Excipients | Route of Administration | Absolute Bioavailability |
|---|---|---|---|
| CorA | Propylene glycol, Kolliphor HS15, PBS Buffer | IV | set to 100% |
| CorA | PVP/VA | PO | 19% |
| CorA | / | PO | 11% |

Figure 8 shows the bioavailability after the oral administration of corallopyronin A embedded in polyvinylpyrrolidone/vinylacetate copolymer (CorA-PVP/VA-ASD) compared to neat corallopyronin A (CorA) (2-compartment model) and further compared to the results for corallopyronin A embedded in polyvinylpyrrolidone (CorA-PVP-ASD) and as determined in example 6.2.

A significant increase of the absorption after oral administration for corallopyronin A embedded in PVP/VA and, therefore, an increase in bioavailability of the amorphous solid dispersions of corallopyronin A was determined. The formulation was able to increase the fraction absorbed and, therefore, the bioavailability. The comparison showed better results for PVP compared to PVP/VA, whether these results will translate into a better pharmacodynamic efficacy, or if PVP/VA will demonstrate e.g., a slower but longer release and therefore a better efficacy needs to be evaluated during clinical studies.

In summary, the results are in line with the *in vitro* findings and the formulation was found very advantageously for the administration of corallopyronin A regarding enhancement of dissolution, solubility, and stability.

Reference

Example 14: Manufacture of a solid corallopyronin A formulation by adsorption to mesoporous silica

Corallopyronin A (10 mg) was adsorbed to mesoporous silica (Syloid® XDP 3050, W. R. Grace & Co.-Conn. Europe) as described in reference example 5. The formulations were prepared and analyzed in triplicate (dissolutions 1, 2, 3) as summarized in table 9.

Table 9: Composition of solid formulations of CorA on mesoporous silica

| Compounds | Dissolution 1 Amounts | Dissolution 2 Amounts | Dissolution 3 Amounts |
|---|---|---|---|
| CorA | 10.2 mg | 10.3 mg | 10.0 mg |
| EtOH | 100 μL | 100 μL | 100 μL |
| Syloid® XDP 3050 | 49.6 mg | 51.1 mg | 50.4 mg |

Reference

Example 15: Manufacture of a liquisolid corallopyronin A formulation by adsorption to mesoporous silica

For the manufacture of the mesoporous silica based liquisolid formulation, 10 mg corallopyronin A and 50 μL propylene carbonate (≥ 99.7%, Carl Roth GmbH + Co. KG) were mixed in a 10 mL glass vial and dissolved by using an ultrasonic bath (Sonorex Digitec, Bandelin electronic GmbH & Co. KG, Berlin, Germany) for about 5 min at a temperature of ≤25 °C until the corallopyronin A was fully dissolved in propylene carbonate. The mesoporous silica (Syloid® XDP 3050, W. R. Grace & Co.-Conn. Europe) was added and mixed with a metal spatula until the solution was fully adsorbed by the silica. The formulations were prepared and analyzed in triplicate (dissolutions 1, 2, 3) as summarized in table 10.

Table 10: Composition of liquisolid formulations of CorA on mesoporous silica

| Compounds | Dissolution 1 Amounts | Dissolution 2 Amounts | Dissolution 3 Amounts |
|---|---|---|---|
| CorA | 10.6 mg | 10.0 mg | 10.1 mg |
| PC | 50 μL | 50 μL | 50 μL |
| Syloid® XDP 3050 | 75.2 mg | 75.1 mg | 76.1 mg |

Reference

Example 16: Biphasic dissolution test of the solid and liquisolid formulations of corallopyronin A on mesoporous silica

The performance of the formulations of Reference Examples 14 and 15 compared to neat corallopyronin A were evaluated via *in vitro* biphasic dissolution apparatus BiPHa+ as described in Example 6 and Reference Example 12.
The following table 11 summarises the biphasic dissolution results for the solid and liquisolid formulations of corallopyronin A on mesoporous silica.

Table 11: Composition of solid and liquisolid formulations of CorA on mesoporous silica

| API | Excipients | Maximum Partitioned Drug in the Organic Phase (%) |
|---|---|---|
| CorA | / | 8 |
| CorA | (Ethanol) Syloid® XDP 3050 | 52 |
| CorA | Propylene Carbonate Syloid® XDP 3050 | 86 |

As can be seen from table 11, both, solid and liquisolid formulations of CorA on mesoporous silica, provided a great increase in the amount of corallopyronin A in the organic phase, which is a surrogate parameter for the fraction absorbed *in vivo*. The increased dissolution and solubility of CorA is assumed to result in an increased bioavailability *in vivo*.

**Claims**

1.   A solid formulation comprising corallopyronin A, wherein the formulation comprises an amorphous solid dispersion of corallopyronin A embedded in a water-soluble polymer selected from the group of polyvinylpyrrolidone and polyvinylpyrrolidone/vinylacetate copolymer.

2.   The formulation according to claim 1, wherein the water-soluble polymer comprises corallopyronin A in an amount in a range of ≥ 10 weight% to ≤ 50 weight%, preferably in an amount in a range of ≥ 15 weight% to ≤ 40 weight%, further preferred in an amount in a range of $\geq 20$ weight% to $\leq 30$ weight%, based on a weight of 100 weight% of the water-soluble polymer and corallopyronin A.

3.  The formulation according to claim 1 or 2, wherein the formulation comprises corallopyronin A embedded in polyvinylpyrrolidone.

4.  The formulation according to any of the preceding claims, wherein the formulation is a solid oral formulation.

5.  The formulation according to any of the preceding claims, wherein the formulation is formulated into a capsule, a tablet, granules, pills, pellets or micro-tablets.

6.  A pharmaceutical solid dosage form, comprising the solid formulation according to claims 1 to 5.

7.  The pharmaceutical solid dosage form according to claim 6, wherein the solid dosage form is selected from a capsule, a tablet, granules, pills, pellets or micro-tablets.

8.  A process of manufacturing a solid formulation according to any of claims 1 to 5, comprising the steps of:

    a) dispersing, dissolving or otherwise introducing corallopyronin A into an organic solvent to form a liquid mixture,
    b) selecting a water-soluble polymer from the group of polyvinylpyrrolidone and polyvinylpyrrolidone/vinylacetate copolymer,
    c) admixing the liquid mixture of step a) and the water-soluble polymer of step b), and
    d) optionally removing excess solvent from the mixture obtained in step c) to form a solid formulation.

9.  The process of claim 8, wherein the organic solvent is selected from ethanol, methanol, isopropanol, dichloromethane, acetone, tert-butanol, propylene carbonate, a C6 to C12 triglyceride, preferably a C8 or C10 triglyceride, a polymer which is liquid at ambient temperature such as a polyethylene glycol, preferably selected from PEG 400, PEG 300 and PEG 200, or mixtures thereof.

10. The process according to claims 8 or 9, wherein in a further step e) the solid formulation is formed into a capsule, a tablet, granules, pills, pellets or micro-tablets.

11. A solid formulation comprising corallopyronin A or a pharmaceutical solid dosage form obtained by the process according to claims 8 to 10.


**Patentansprüche**

1.  Feste Formulierung umfassend Corallopyronin A, wobei die Formulierung eine amorphe feste Dispersion von Corallopyronin A eingebettet in ein wasserlösliches Polymer ausgewählt aus der Gruppe von Polyvinylpyrrolidon und Polyvinylpyrrolidon/Vinylacetat-Copolymer umfasst.

2.  Formulierung gemäß Anspruch 1, wobei das wasserlösliche Polymer Corallopyronin A in einer Menge im Bereich von $\geq 10$ Gew.-% bis $\leq 50$ Gew.-%, vorzugsweise in einer Menge im Bereich von $\geq 15$ Gew.-% bis $\leq 40$ Gew.-%, weiter bevorzugt in einer Menge im Bereich von $\geq 20$ Gew.-% bis $\leq 30$ Gew.-%, bezogen auf ein Gewicht von 100 Gew.-% an wasserlöslichem Polymer und Corallopyronin A, umfasst.

3.  Formulierung gemäß Anspruch 1 oder 2, wobei die Formulierung Corallopyronin A eingebettet in Polyvinylpyrrolidon umfasst.

4.  Formulierung gemäß einem der vorstehenden Ansprüche, wobei die Formulierung eine feste orale Formulierung ist.

5.  Formulierung gemäß einem der vorstehenden Ansprüche, wobei die Formulierung zu einer Kapsel, einer Tablette, Granulaten, Pillen, Pellets oder Mikrotabletten formuliert ist.

6.  Pharmazeutische feste Darreichungsform, umfassend die feste Formulierung gemäß den Ansprüchen 1 bis 5.

7.  Pharmazeutische feste Darreichungsform gemäß Anspruch 6, wobei die feste Darreichungsform ausgewählt ist aus einer Kapsel, einer Tablette, Granulaten, Pillen, Pellets oder Mikrotabletten.

8. Verfahren zur Herstellung einer festen Formulierung gemäß einem der Ansprüche 1 bis 5, umfassend die folgenden Schritte:

a) Dispergieren, Auflösen oder anderweitiges Einbringen von Corallopyronin A in ein organisches Lösungsmittel, um eine flüssige Mischung zu bilden,
b) Auswählen eines wasserlöslichen Polymers aus der Gruppe von Polyvinylpyrrolidon und Polyvinylpyrrolidon/Vinylacetat-Copolymer,
c) Vermischen der flüssigen Mischung aus Schritt a) und des wasserlöslichen Polymers aus Schritt b) und
d) optional Entfernen von überschüssigem Lösungsmittel aus der in Schritt c) erhaltenen Mischung, um eine feste Formulierung zu bilden.

9. Verfahren nach Anspruch 8, wobei das organische Lösungsmittel ausgewählt ist aus Ethanol, Methanol, Isopropanol, Dichlormethan, Aceton, tert-Butanol, Propylencarbonat, einem C6- bis C12-Triglycerid, vorzugsweise einem C8- oder C10-Triglycerid, einem bei Umgebungstemperatur flüssigen Polymer wie Polyethylenglykol, vorzugsweise ausgewählt aus PEG 400, PEG 300 und PEG 200, oder Mischungen davon.

10. Verfahren nach Anspruch 8 oder 9, wobei in einem weiteren Schritt e) die feste Formulierung zu einer Kapsel, einer Tablette, Granulaten, Pillen, Pellets oder Mikrotabletten geformt wird.

11. Feste Formulierung umfassend Corallopyronin A oder pharmazeutische feste Darreichungsform erhalten durch das Verfahren nach Anspruch 8 bis 10.

**Revendications**

1. Formulation solide comprenant de la corallopyronine A, dans laquelle la formulation comprend une dispersion solide amorphe de corallopyronine A incorporée dans un polymère hydrosoluble choisi dans le groupe de la polyvinylpyrrolidone et d'un copolymère de polyvinylpyrrolidone/acétate de vinyle.

2. Formulation selon la revendication 1, dans laquelle le polymère hydrosoluble comprend
de la corallopyronine A en une quantité dans une plage de ≥ 10 % en poids à ≤ 50 % en poids, de préférence en une quantité dans une plage de ≥ 15 % en poids à ≤ 40 % en poids, de préférence encore en une quantité dans une plage de ≥ 20 % en poids à ≤ 30 % en poids, sur la base d'un poids de 100 % en poids du polymère hydrosoluble et de la corallopyronine A.

3. Formulation selon la revendication 1 ou 2, dans laquelle la formulation comprend
de la corallopyronine A incorporée dans la polyvinylpyrrolidone.

4. Formulation selon l'une quelconque des revendications précédentes, dans laquelle la formulation est une formulation orale solide.

5. Formulation selon l'une quelconque des revendications précédentes, dans laquelle la formulation est formulée en une capsule, un comprimé, des granules, des pilules, des pastilles ou des microcomprimés.

6. Forme galénique solide pharmaceutique, comprenant la formulation solide selon les revendications 1 à 5.

7. Forme galénique solide pharmaceutique selon la revendication 6, dans laquelle la forme galénique solide est choisie parmi une capsule, un comprimé, des granules, des pilules, des pastilles ou des microcomprimés.

8. Procédé de fabrication d'une formulation solide selon l'une quelconque des revendications 1 à 5, comprenant les étapes de :

a) dispersion, dissolution ou sinon introduction de la corallopyronine A dans un solvant organique, pour former un mélange liquide,
b) sélection d'un polymère hydrosoluble dans le groupe d'une polyvinylpyrrolidone et d'un copolymère de polyvinylpyrrolidone/acétate de vinyle,

c) mélange du mélange liquide de l'étape a) et du polymère hydrosoluble de l'étape b), et

d) éventuellement élimination de solvant en excès du mélange obtenu dans l'étape c) pour former une formulation solide.

9. Procédé selon la revendication 8, dans lequel le solvant organique est choisi parmi l'éthanol, le méthanol, l'isopropanol, le dichlorométhane, l'acétone, le tertio-butanol, le carbonate de propylène, un triglycéride en C6 à C12, de préférence un triglycéride en C8 ou C10, un polymère liquide à température ambiante tel qu'un polyéthylène glycol, de préférence choisi parmi PEG 400, PEG 300, PEG 200, ou leurs mélanges.

10. Procédé selon la revendication 8 ou 9, dans lequel, dans une étape supplémentaire e), la formulation solide est mise sous forme d'une capsule, d'un comprimé, de granulés, de pilules, de pastilles ou de microcomprimés.

11. Formulation solide comprenant de la corallopyronine A ou une forme galénique solide pharmaceutique obtenue par le procédé selon les revendications 8 à 10.

Figure 1

Figure 2

Figure 3

Figure 4

Figure 5

Figure 6

Figure 7

Figure 8

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2520293 A1

**Non-patent literature cited in the description**

- **A. DENNINGER et al.** *Pharmaceutics*, 2020, vol. 12, 237